(19)

Europäisches
Patentamt
European
Patent Office
Office européen
des brevets

(11) **EP 4 282 950 A1**

(12) **EUROPEAN PATENT APPLICATION**
published in accordance with Art. 153(4) EPC

(43) Date of publication:
**29.11.2023 Bulletin 2023/48**

(21) Application number: **21920502.8**

(22) Date of filing: **08.06.2021**

(51) International Patent Classification (IPC):
*C12N 1/20* (2006.01)       *C12N 1/02* (2006.01)
*A61K 35/744* (2015.01)      *A61K 45/06* (2006.01)
*A61P 35/00* (2006.01)

(86) International application number:
**PCT/CN2021/098848**

(87) International publication number:
**WO 2022/156119 (28.07.2022 Gazette 2022/30)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **19.01.2021 CN 202110072294
21.01.2021 CN 202110085233**

(71) Applicant: **Moon (Guangzhou) Biotech Co., Ltd.
Guangzhou, Guangdong 510535 (CN)**

(72) Inventors:
• **LI, Baoxia
  Guangzhou, Guangdong 510535 (CN)**
• **LIN, Quansheng
  Guangzhou, Guangdong 510535 (CN)**

• **XIAN, Yibo
  Guangzhou, Guangdong 510535 (CN)**
• **JIANG, Xianzhi
  Guangzhou, Guangdong 510535 (CN)**
• **LIANG, Jiening
  Guangzhou, Guangdong 510535 (CN)**
• **LIU, Zhenzhen
  Guangzhou, Guangdong 510535 (CN)**
• **CHEN, Sheng
  Guangzhou, Guangdong 510535 (CN)**
• **SU, Xue
  Guangzhou, Guangdong 510535 (CN)**

(74) Representative: **Plasseraud IP
66, rue de la Chaussée d'Antin
75440 Paris Cedex 09 (FR)**

Remarks:
The complete document including Reference
Table(s) and the Sequence Listing(s) can be
downloaded from the EPO website

(54) **ENTEROCOCCUS FAECIUM, DRUG FOR PREVENTING OR TREATING TUMOR, AND APPLICATION**

(57)     Provided are Enterococcus lactis, a drug for preventing or treating tumor, and use thereof, relating to the technical field of biomedicines. Novel Enterococcus lactis is separated and chosen, and the Enterococcus lactis has an important influence on the effect of tumor immunotherapy for cancer patients. Gut microorganisms and immune cells can interact with each other to jointly regulate the human immune system. The provided Enterococcus lactis can induce the differentiation and maturation of iDC (immature DC) cells, and mature DC can effectively activate T cells and increase the infiltration of tumor infiltrating lymphocytes having anti-tumor activity, thereby killing tumor cells. In addition, the provided Enterococcus lactis can induce the differentiation of M0 type macrophages into M1 or M2 type cells.

FIG. 1

EP 4 282 950 A1

## Description

### Cross-Reference to Related Applications

The disclosure claims the priority of CN Application: CN202110072294.9, filed before the Chinese Patent Office on January 19, 2021, entitled "Enterococcus lactis, drug for preventing or treating tumors and use thereof ", and CN Application: CN202110085233.6, filed before the Chinese Patent Office on January 21, 2021, entitled "Enterococcus lactis, drug for preventing or treating tumors and use thereof", which are herein incorporated by reference in their entirety.

### Field

The disclosure relates to the field of medical biotechnology, specifically, to Enterococcus lactis, drug for preventing or treating tumors and use thereof.

### Background

Tumors are the result of immune escape. Although tumor immunotherapy has progressed rapidly, standard therapies for various cancers are constantly being rewritten. Moreover, a single drug does not have a high efficacy, there are primary and secondary drug resistance, and the number of population obtaining the benefit is small. Improving the therapeutic effect and benefiting more tumor patients are clinical problems that need to be solved urgently. PD-1/PD-L1 immunotherapy is a new type of anti-tumor immunotherapy that has attracted much attention and been widely studied in the world. It aims at making full use of the human body's own immune system to resist and fight against tumors by blocking the PD-1/PD-L1 signaling pathway to cause tumor cell death, and it has the potential to treat various types of tumors and substantially improve the overall survival of patients.

At present, the existing immunotherapy has some problems such as toxic and side effects and treatment failure. The introduction of gut microorganisms for immunotherapy treatment is a new intervention method.

In view of the above, the disclosure is proposed.

### Summary

The disclosure provides a use of Enterococcus lactis in the preparation of a tumor inhibitor. In some embodiments, the tumor optionally comprises at least one of the solid tumors from the group consisting of colon cancer, rectal cancer, colorectal cancer, liver cancer, pancreatic cancer, breast cancer, kidney cancer, fibrosarcoma, lung cancer and cholangiocarcinoma.

In alternative embodiments, the tumor further comprises the solid tumors from the group consisting of ovarian cancer, cervical cancer, prostate cancer, bladder cancer, head and neck cancer, myeloma, lymphoma, brain tumor, spinal tumor, esophageal cancer, oropharyngeal cancer, laryngeal cancer, colorectal cancer, melanoma, neuroendocrine cancer, CNS cancer, non-Hodgkin's lymphoma, hematological malignancies, renal tumors, neuroblastoma, thermostatic sarcoma, Ewing's family sarcoma, retinoma, diffuse large cell lymphoma, and advanced CD70+ cancer.

In alternative embodiments, the Enterococcus lactis is deposited under No. GDMCC NO: 61121 in the Guangdong Microbial Culture Collection Center, named as Enterococcus lactis MNC-168.

In alternative embodiments, colony culture characteristics of the Enterococcus lactis are: the colony is white, round, having a moist surface, opaque, and having neat edges after culturing for 24 hours in a bacterial culture medium; and the bacteria are ellipsoid, 0.7-1.0μm × 0.8-1.3μm, arranged singly or in pairs under a microscope, and Gram positive.

In alternative embodiments, the bacterial culture medium is MRS medium.

The disclosure provides a drug for preventing or treating tumors, wherein the drug comprises Enterococcus lactis.

In alternative embodiments, the drug for preventing or treating tumors further comprises pharmaceutically acceptable additives or adjuvants. In alternative embodiments, the formulation of the pharmaceutical composition is selected from the group consisting of tablet, pill, powder, suspension, gel, emulsion, cream, granule, nanoparticle, capsule, suppositorie, spray and injection.

In alternative embodiments, the pharmaceutically acceptable additive comprises one or more from the group consisting of filler, diluent, wetting agent, disintegrant, glidant, lubricant, binder, and colourant.

In alternative embodiments, the pharmaceutically acceptable adjuvant comprises one or more from the group consisting of cyclodextrin, starch, sucrose, lactose, hydroxypropylcellulose, hydroxypropylmethylcellulose, magnesium stearate, talcum, iron oxide, and sodium starch glycolate.

In alternative embodiments, the Enterococcus lactis is deposited under No. GDMCC NO: 61121 in the Guangdong Microbial Culture Collection Center, named as Enterococcus lactis MNC-168.

The disclosure provides a pharmaceutical composition, wherein the pharmaceutical composition comprises the drug for preventing or treating tumors.

In alternative embodiments, the pharmaceutical composition further comprises a drug for use in combination. In alternative embodiments, the drug for use in combination is at least one of the drugs from the group consisting of chemotherapeutic agent, photosensitizer, photothermal agent, antibody of inhibitory second signaling molecule, inhibitor of inhibitory second signaling molecule, PD-L1 inhibitor, and PD-1/PD-L1 monoclonal antibody.

In alternative embodiments, the drug for use in combination is at least one of the drugs from the group consisting of chemotherapeutic agent, photosensitizer, photothermal agent, antibody of inhibitory signaling pathway molecule, inhibitor of inhibitory signaling pathway molecule, PD-L1 inhibitor, and PD-1/PD-L1 monoclonal antibody.

In alternative embodiments, the inhibitor of inhibitory second signaling molecule comprises at least one of the signaling molecules from the group consisting of PD-1 and CTLA-4.

In alternative embodiments, the inhibitory signaling pathway molecule comprises at least one of the signaling molecules from the group consisting of PD-1 and CTLA-4.

In alternative embodiments, the antibody of inhibitory second signaling molecule or the inhibitor of inhibitory second signaling molecule comprises at least one of the antibodies from the group consisting of nivolumab, pembrolizumab, tislelizumab, nivolumab injection, pembrolizumab injection, toripalimab injection and sintilimab injection.

In alternative embodiments, the antibody of inhibitory signaling pathway molecule or the inhibitor of inhibitory signaling pathway molecule comprises at least one of the antibodies from the group consisting of nivolumab, pembrolizumab, tislelizumab, nivolumab injection, pembrolizumab injection, toripalimab injection and sintilimab injection.

In alternative embodiments, the PD-L1 inhibitor is selected from durvalumab, atezolizumab or avelumab.

In alternative embodiments, the PD-1/PD-L1 monoclonal antibody is selected from pembrolizumab or nivolumab.

The disclosure provides an Enterococcus lactis, wherein the Enterococcus lactis is deposited under No. GDMCC NO: 61121 in the Guangdong Microbial Culture Collection Center, named as Enterococcus lactis MNC-168.

In alternative embodiments, the colony culture characteristics of Enterococcus lactis are: the colony is white, round, having a moist surface, opaque, and having neat edges after culturing for 24 hours in a bacterial culture medium; and the bacteria are ellipsoid, 0.7-1.0$\mu$m $\times$ 0.8-1.3$\mu$m, arranged singly or in pairs under a microscope, and Gram positive.

In alternative embodiments, the bacterial culture medium is MRS medium.

The disclosure provides the use of the drug and/or the pharmaceutical composition for the treatment of tumors.

The disclosure provides a method for treating tumors comprising:

administering a therapeutically effective amount of the drug or the pharmaceutical composition to the subject in need thereof.

In an alternative embodiment, said drug or the pharmaceutical composition is administered via at least one from the group consisting of oral, intravenous, subcutaneous, intraperitoneal, rectal, intramuscular, dermal, transdermal, topical, and via any other parenteral route, in a pharmaceutically acceptable formulation.

**Brief description of the drawings**

In order to more clearly illustrate the technical solutions of the embodiments of the disclosure, the accompanying drawings used in the embodiments will be briefly introduced as follows. It should be understood that the following drawings only indicate some embodiments of the disclosure, and therefore should not be regarded as a limitation on the scope. For those skilled in the art, other related drawings can also be obtained from these drawings without creative efforts.

**Figure 1** shows the growth status graph of MNC-168 on the plate;

**Figure 2A** shows the change of cytokine IL-1$\beta$ produced by macrophages and MNC-168 after co-culture at a MOI (bacteria: cells) of 10, wherein control group represents: PBS control group, LPS+INFr represents: M1 macrophage control group, and MNC-168 represents: test group;

**Figure 2B** shows the change of cytokine IL-23 produced by macrophages and MNC-168 after co-culture at a MOI (bacteria: cells) of 10, wherein control group represents: PBS control group, LPS+INFr represents: M1 macrophage control group, and MNC-168 represents: test group;

**Figure 2C** shows the change of cytokine TNF$\alpha$ produced by macrophages and MNC-168 after co-culture at a MOI (bacteria: cells) of 10, wherein control group represents: PBS control group, LPS+INFr represents: M1 macrophage control group, and MNC -168 represents: test group;

**Figure 3A** shows the change of cytokine IL-1$\beta$ produced by Primary PBMC and MNC-168 after co-culture at a MOI (bacteria: cell) of 1, wherein control group represents: PBS control group, and MNC-168 represents: test group;

**Figure 3B** shows the change of the cytokine CXCL9 produced by Primary PBMC and MNC-168 after co-culture at a MOI (bacteria: cell) of 1, wherein the control group represents: PBS control group, and MNC-168 represents: test group;

Figure 4A shows the change of cytokine CXCL9 produced by iDC cells and MNC-168 after co-culture at a MOI (bacteria: cells) of 10, wherein PBS represents: PBS control group, LPS represents: positive control group, and MNC-168 represents: test group;

**Figure 4B** shows the change of cytokine IL-1$\beta$ produced by iDC cells and MNC-168 after co-culture at a MOI (bacteria: cells) of 10, wherein PBS represents: PBS control group, LPS represents: positive control group, and MNC-168 represents: test group;

**Figure 5** shows the survival curve (absorbance value) of MNC-168 in simulated gastric fluid;

**Figure 6** shows the survival curve (absorbance value) of MNC-168 in simulated intestinal fluid;

**Figure 7** shows a graph showing the inhibitory effect of the combination of MNC-168 and PD-1 antibody against colorectal cancer (CT26);

**Figure 8** shows a graph showing the inhibitory effect of the combination of MNC-168 and PD-1 antibody against colorectal cancer (MC38);

**Figure 9** shows MNC-168 reverses the non-response rate of PD-1 antibody; wherein (a) is the average tumor volume curve, and (b) is the tumor volume curve of a single mouse;

**Figure 10** shows that MNC-168 increases lymphocyte infiltration in the tumor; wherein (a) is the result of histochemical staining (CD4, CD8, INF-$\gamma$, and Foxp3), (b) is the result of flow staining (INF-$\gamma$ %/CD4 ), and (c) is the result of flow staining (INF-$\gamma$ %/CD8);

**Figure 11** shows a graph of MNC-168 streak cultured on the MRS medium for 24 hours;

**Figure 12** shows a morphological diagram of MNC-168 under an optical microscope after staining;

**Figure 13** shows the electron micrograph of MNC-168;

**Figure 14** shows the 16S rDNA sequence phylogenetic tree of MNC-168 and related species; and

**Figure 15** shows a CD14 expression graph detected by flow cytometry after THP1 cells are induced by PMA to M0 macrophages, wherein control group represents: PBS control group, and PMA represents: M0 macrophage group.

## DETAILED DESCRIPTION OF THE INVENTION

In order to make the technical solutions and advantages of the embodiments of the disclosure clearer, the technical solutions in the embodiments of the disclosure will be clearly and completely described below.

<u>Definition of Terms</u>

As used herein, "tumor inhibitor" refers to an agent that inhibits the division and growth of tumor cells.

As used herein, "drug for use in combination" refers to the simultaneous or sequential application of one or two or more drugs that are added additionally for the purpose of treatment. As used herein, the term "inhibitory signaling pathway molecule" refers to a signaling molecule that inhibits the immune response.

As used herein, the term "antibody of inhibitory signaling pathway molecule" refers to a protective protein produced by the body due to the stimulation of inhibitory signaling pathway molecule.

As used herein, the term "inhibitor of inhibitory signaling pathway molecule" refers to an agent used for the inhibition produced by an inhibitory signaling pathway molecule.

As used herein, the term "preventing" refers to preventing subject from developing a disorder or reducing the risk of developing a disorder of subject.

As used herein, "treating" can generally refer to treating, healing, and/or ameliorating a disease, disorder, condition, or side effect, or to decreasing in the rate of advancement of a disease, disorder, condition, or side effect. The term also includes within its scope enhancing normal physiological function, palliative treatment, and partial remediation of a disease, disorder, condition, side effect, or symptom thereof.

As used herein, the term "dose" as used herein refers to a daily quantity that is administered to a subject. The daily quantity or dose may be administered all at once or it may be spread out over several administrations throughout a day. The dose may be by administered by any known method, such as orally, by injection and the like. As used herein, the term "subject" refers to a vertebrate, preferably a mammal, most preferably a human. Mammals include, but are not limited to, murines, simians, humans, livestocks, racing animals, and pets. Tissues, cells and progeny of biological entities obtained in vivo or cultured in vitro are further encompassed.

As used herein, the term "therapeutically effective amount" refers to an amount of an agent that is sufficient to effect beneficial or desired results. The therapeutically effective amount may vary depending upon one or more of: the subject and disease condition being treated, the weight and age of the subject, the severity of the disease condition, the manner of administration and the like, which can readily be determined by one of ordinary skill in the art.

As used herein, the term "MO" or "MO type" refers to the stage of unpolarized macrophages, i.e., the M0 phase, for example, the terms "MO type macrophages" and "MO macrophages" refer to the macrophages in the unpolarized state, that is, the cells of M0 phase.

The disclosure provides an embodiment, and provides an Enterococcus lactis, a drug for preventing or treating tumors and use thereof to solve the above technical problems.

It is believed that without being bound of theory, based on the existing technical problems, the disclosure isolates and screens a strain of Enterococcus lactis, which can inhibit the growth rate of tumors and can be used for preventing or treating tumors. In addition, the Enterococcus lactis can also be used in combination with other tumor inhibitors.

This disclosure is achieved by:

I. Use of Enterococcus lactis in the preparation of a tumor inhibitor (for the prevention or treatment of tumors)

The disclosure provides an embodiment, and provides the use of Enterococcus lactis in the preparation of a tumor inhibitor; the tumor optionally comprises at least one of the solid tumors from the group consisting of colon cancer, rectal cancer, colorectal cancer, liver cancer, pancreatic cancer, breast cancer, kidney cancer, fibrosarcoma, lung cancer and cholangiocarcinoma.

In alternative embodiments, the tumor further comprises the solid tumors from the group consisting of ovarian cancer, cervical cancer, prostate cancer, bladder cancer, head and neck cancer, myeloma, lymphoma, brain tumor, spinal tumor, esophageal cancer, oropharyngeal cancer, laryngeal cancer, colorectal cancer, melanoma, neuroendocrine carcinoma, CNS carcinoma, non-hodgkin lymphoma, hematological malignancies, kidney neoplasms, neuroblastoma, thermostatous sarcoma, Ewing's family sarcoma, retinoma, diffuse large cell lymphoma, and advanced cd70+ cancer.

Alternatively, the disclosure provides the use of Enterococcus lactis in the preparation of colorectal cancer inhibitor.

Alternatively, the above-mentioned Enterococcus lactis is deposited under No. GDMCC NO: 61121 in the Guangdong Microbial Culture Collection Center at Guangdong Institute of Microbiology, 5th Floor, Building 59, No. 100 Xianlie Middle Road, Guangzhou on August 5, 2020, named as Enterococcus lactis MNC-168. The classification name is Enterococcus lactis.

In alternative embodiments of the use of the disclosure, the colony culture characteristics of the above-mentioned Enterococcus lactis are: the colony is white, round, moist surface, opaque, and having neat edges after culturing for 24 hours in a bacterial culture medium; and the bacteria are ellipsoid, $0.7\text{-}1.0\mu m \times 0.8\text{-}1.3\mu m$, arranged singly or in pairs under a microscope, and Gram positive;

Alternatively, the bacterial culture medium is MRS medium.

II. Drugs for preventing or treating tumors.

The disclosure also provides an embodiment, and provides a drug for preventing or treating tumors, wherein the drug comprises the Enterococcus lactis.

In alternative embodiments of the use of the disclosure, the above-mentioned drug for preventing or treating tumors further comprises pharmaceutically acceptable additives or adjuvants.

In alternative embodiments, the pharmaceutically acceptable additive comprises one or more from the group consisting of filler, diluent, wetting agent, disintegrant, glidant, lubricant, binder, and colourant.

In alternative embodiments, the pharmaceutically acceptable adjuvant comprises one or more from the group consisting of cyclodextrin, starch, sucrose, lactose, hydroxypropylcellulose, hydroxypropylmethylcellulose, magnesium stearate, talcum, iron oxide, and sodium starch glycolate.

In alternative embodiments, the formulation of the pharmaceutical composition is selected from the group consisting of tablet, pill, powder, suspension, gel, emulsion, cream, granule formulation, nanoparticle, capsule, suppositorie, spray and injection.

In other alternative embodiments, the active ingredient of above-mentioned drug for preventing or treating tumors includes, but is not limited to, Enterococcus lactis, and may further comprise other drugs or antibodies for preventing or treating tumors.

Enterococcus lactis is deposited under No. GDMCC NO: 61121 in the Guangdong Microbial Culture Collection Center, named as Enterococcus lactis MNC-168.

III. Pharmaceutical composition

The disclosure also provides a pharmaceutical composition, wherein the pharmaceutical composition comprises the above-mentioned drugs for preventing or treating tumors.

Alternatively, the above-mentioned pharmaceutical composition further comprises a drug for use in combination, and the drug for use in combination is at least one of the drugs from the group consisting of: chemotherapeutic agent, photosensitizer, photothermal agent, antibody of inhibitory second signaling molecule, inhibitor of inhibitory second signaling molecule, PD-L1 inhibitor, and PD-1/PD-L1 monoclonal antibody.

In alternative embodiments, the drug for use in combination is at least one of the drugs from the group consisting of: chemotherapeutic agent, photosensitizer, photothermal agent, antibody of inhibitory signaling pathway molecule, inhibitor of inhibitory signaling pathway molecule, PD-L1 inhibitor, and PD-1/PD-L1 monoclonal antibody. The inhibitory signaling pathway molecule comprises inhibitory second signaling pathway molecule.

In alternative embodiments of the use of the disclosure, the above-mentioned inhibitor of inhibitory second signaling molecule comprises at least one of the signaling molecules from the group consisting of PD-1 and CTLA-4. In other embodiments, the above mentioned inhibitor of inhibitory second signaling molecule may be PD-1; CTLA-4; PD-1 and CTLA-4.

In alternative embodiments of the use of the disclosure, the inhibitory signaling pathway molecule comprises at least one of the signaling molecules from the group consisting of PD-1 and CTLA-4. In other embodiments, the above mentioned inhibitory signaling pathway molecule may be PD-1; CTLA-4; PD-1 and CTLA-4.

It is believed, without being bound of theory, that the above-mentioned inhibitor of inhibitory second signaling molecule refers to a signaling molecule that inhibits the immune response. It is believed, without being bound of theory, that the above-mentioned "inhibitory signaling pathway molecule" refers to a signaling molecule that inhibits the immune response.

In alternative embodiments of the use of the disclosure, the above-mentioned antibody of inhibitory second signaling molecule or the inhibitor of inhibitory second signaling molecule comprises at least one of the following antibodies: nivolumab, pembrolizumab, tislelizumab, nivolumab injection (Opdivo), pembrolizumab injection (Keytruda), toripalizumab injection (Tuoyi JS001) and sintilimab injection ( Tyvyt IBI308).

In alternative embodiments of the use of the disclosure, the antibody of inhibitory signaling pathway molecule or the inhibitor of inhibitory signaling pathway molecule comprises at least one of the following antibodies: nivolumab, pembrolizumab, tislelizumab, nivolumab injection, pembrolizumab injection, toripalimab injection and sintilimab injection.

In alternative embodiments of the use of the disclosure, the above mentioned PD-L1 inhibitor is selected from durvalumab, atezolizumab or avelumab.

In alternative embodiments of the use of the disclosure, the above mentioned PD-1/PD-L1 monoclonal antibody is selected from pembrolizumab or nivolumab.

In other alternative embodiments, Enterococcus lactis provided by the disclosure can be combined with anyone that can inhibit the occurrence of immune response for combined tumor treatment.

The disclosure provides the use of the drug and/or the pharmaceutical composition for the treatment of tumors.

The disclosure provides a method for treating tumors comprising: administering a therapeutically effective amount of the drug or the pharmaceutical composition to the subject in need thereof.

In alternative embodiments, the tumor comprises at least one of the solid tumors from the group consisting of colon cancer, rectal cancer, colorectal cancer, liver cancer, pancreatic cancer, breast cancer, kidney cancer, fibrosarcoma, lung cancer, and cholangiocarcinoma.

In an alternative embodiment, said drug or the pharmaceutical composition is administered via at least one from the group consisting of oral, intravenous, subcutaneous, intraperitoneal, rectal, intramuscular, dermal, transdermal, topical, and via any other parenteral route, in a pharmaceutically acceptable formulation.

The disclosure has the following beneficial effects:

The disclosure isolates and screens a new Enterococcus lactis that has an important influence on the effect of tumor immunotherapy for cancer patients. Gut microorganisms can interact with immune cells to jointly regulate the human immune system. Studies have indicated that the Enterococcus lactis provided by the disclosure can induce the differentiation and maturation of iDC (immature DC) cells, and the mature DC can effectively activate T cells, thereby killing tumor cells. In addition, the Enterococcus lactis provided by the disclosure can induce the differentiation of M0 type macrophages into M1 or M2 type cells, thereby significantly increasing the secretion of IL-1$\beta$, IL-23 and TNF$\alpha$. The Enterococcus lactis provided by the disclosure can be used in the preparation of a tumor inhibitor and the corresponding drug, thereby realizing the prevention or treatment of tumor.

**Example**

In order to make the purpose, technical solutions and advantages of the embodiments of the disclosure clearer, the technical solutions in the embodiments of the disclosure will be clearly and completely described below. Those embodiments that do not indicate the specific conditions are carried out according to the conventional conditions or the conditions suggested by the manufacturer. The reagents or instruments used which are not indicated by the manufacturer are all conventional products that can be purchased from the market.
The features and performances of the disclosure will be further described by reference to the following examples:

Example 1

The example provides an Enterococcus lactis, and its isolation, purification, and colony identification were as follows.
Isolation and purification of strain MNC-168.

(1) Physiological saline was dispensed into sterile 10ml centrifuge tubes in a biological safety cabinet. The anaerobic blood plate and sterile physiological saline were transferred into an anaerobic workplatform (H35 Hypoxystation, Whitley) 24 hours in advance, and 5-7 sterile glass beads were poured into the solidified anaerobic blood plate for later use. Anaerobic blood plates were purchased from Jiangmen Carin Trading Co., Ltd., Bio-Caring.

(2) After an employee in Moon (Guangzhou) Biotechnology Co., Ltd. (donor number 403051) signing an informed consent form for feces collection with the company, 1g of his fresh fecal sample was sampled and shaken with a vortex shaker on an anaerobic operating table for lmin, mix well. 1mL sample was pipetted into 9mL physiological saline, mixed well to obtain a $10^{-1}$ dilution, which was sequentially diluted to a $10^{-6}$ dilution for coating on a plate.

(3) 100$\mu$L of the $10^{-6}$ dilution in step (2) was pipetted into the anaerobic blood plate, and spread evenly with glass beads. The glass beads were discard after the surface of the plate was dry, and the plate was incubated anaerobically at 37°C on the anaerobic workplatform for 3-5 days.

(4) The growth status of the isolated strains was observed, and a single colony was picked for plate streak purification and culture by a sterilized toothpick. A pure strain was analyzed for 16S rRNA gene, thereby preliminarily determining their taxonomic status.

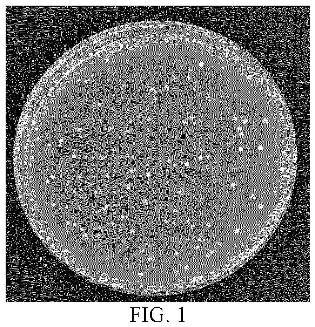

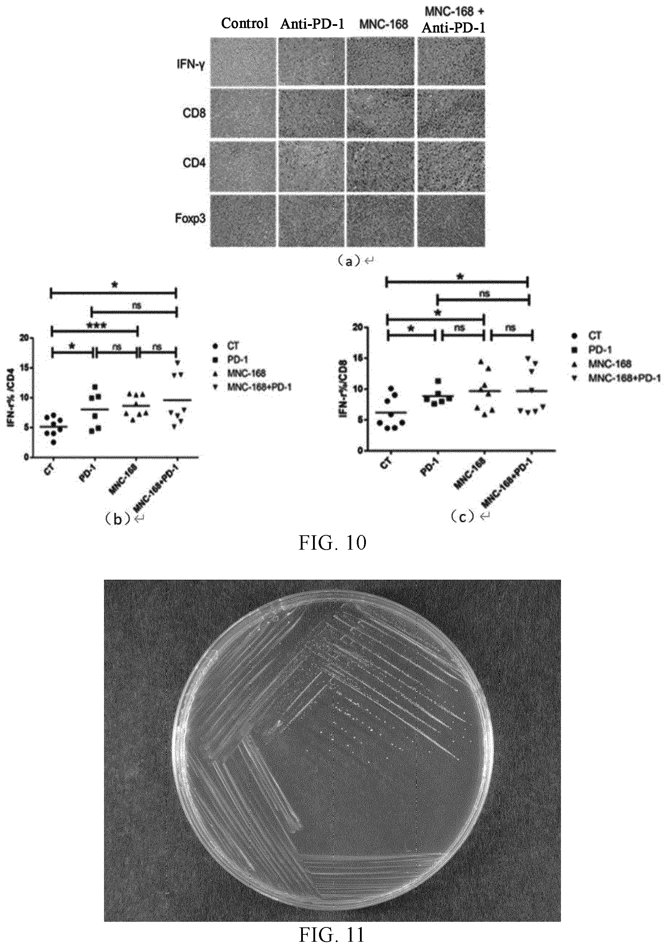

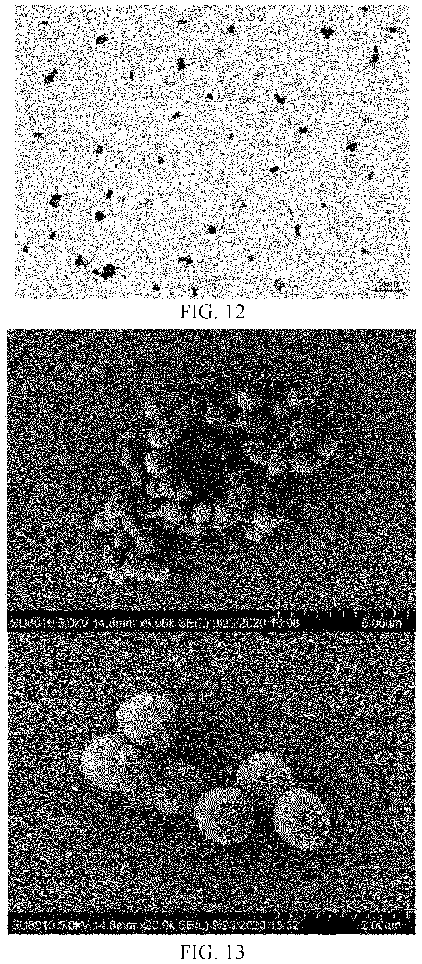

The isolated strain was named as MNC-168, and its 16s RNA sequence is shown in SEQ ID NO.1.
The plate graph of the isolated strain streak cultured on the MRS medium is shown in Figure 11. Figure 11 was a graph of MNC-168 cultured on the MRS medium at 36 °C for 24 hours. The colony was white, round, having a moist surface, opaque, and having neat edges.
The growth status of the MNC-168 strain on the plate is shown in Figure 1.
The morphology of MNC-168 after staining under the optical microscope is shown in Figure 12. Under the microscope, the bacteria were ellipsoid, 0.7-1.0$\mu$m $\times$ 0.8-1.6$\mu$m, arranged singly or in pairs, and Gram-positive bacteria after cultured in MRS medium at 36 °C for 24 hours.
The morphology of MNC-168 under the electron microscope is shown in Figure 13. It can be seen from Figure 13 that the isolated MNC-168 is ellipsoid, in size of 0.7-1.0 $\mu$m $\times$ 0.8-1.6 $\mu$m, and arranged singly or in pairs.
The physiological and biochemical characteristics of MNC-168 are shown in Table 1. In the example, API 20Strep (BioMérieux, CN2060025) biochemical identification reagent strip was used for the physiological and biochemical identification of microorganisms.
Table 1 Physiological and biochemical characteristics of MNC-168.

Explanation of symbols: "+" represents positive; and "-" represents negative.

The antibiotic susceptibility test results of MNC-168 are shown in Table 2. It can be seen from Table 2 that MNC-168 has a certain resistance to erythromycin and rifampicin.

Table 2 Antibiotic susceptibility test for MNC-168.

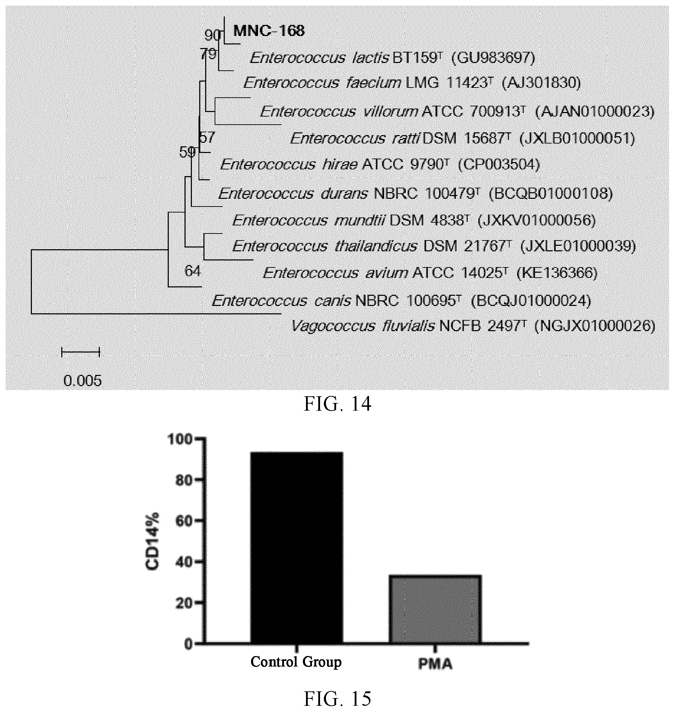

Figure 14 is the 16S rDNA sequence phylogenetic tree of MNC-168 and related species. MEGA5.0 software was used to display the 16S rDNA sequence phylogenetic tree of "MNC-168RCB" and related species by the adjacent junction method, and 1000 times similarity calculations were repeated. Only the Bootstrap values greater than 50% of the value in the phylogenetic tree nodes were showed in the Figure, and the superscript "T" indicated the model strain.

Example 2

In the example, the genome of the original strain MNC-168 isolated and obtained in Example 1 was prepared, sequenced, assembled and analyzed.

The genome of the original strain of MNC-168 was fragmented by ultrasonic method (Covaris LE220R-plus, FastPreP-245G) with a fragmentation length range of ~350bp, and then a standard DNA library construction kit (NEB NEBNext® Ultra™) was used to construct an Illumina sequencing library. The constructed sequencing library was subjected to paired-end 150 bp sequencing by using NovaSeq (Illumina). 2.85Gbp data was obtained by sequencing, of which Q20 accounted for: 96.605%.

The original sequencing data of the genome was filtered by using fastp (version: 0.20.0) with filtering parameters: "-q 15-150". The filtered raw data was subjected to genome assembly by using SPAdes (version: v3.13.1) with assembly parameters "--isolate--cov-cutoff 10". The gene obtained in genome assembly had a total length of 2.84 Mbp, an N50 length of 79.90kbp, and a GC content of 38.17%. The model strain with the highest genome similarity was: Enterococcus lactis, in which the average nucleotide identity (ANI) was 98.36%, and the gene coverage was 86.98%. Therefore, it can be identified as the same strain of Enterococcus lactis.

Genome genes were predicted and analyzed by using the prokaryotic analysis software genome annotation process prokka (version: 1.14.5) with parameters "-gcode 11--evalue 1e-09-coverage 80". A total of 2769 CDS sequences were predicted, which had an average CDS sequence length of 873bp.

Potential antibiotic resistance genes in the genome were analyzed by using the RGI workflow (version: 4.2.2), wherein the antibiotic resistance gene database was CARD (version: 3.0.0, https://card.mcmaster.ca/analyze/rgi). Detailed comparison information was shown in Table 3.

Table 3 List of resistance gene information.

NCBI blastp (version: 2.9.0) was used to compare the virulence factor database VFDB (virulence factor database, http://www.mgc.ac.cn/ cgi-bin/VFs/v5/main.cgi, updated on September 13, 2019) for the analysis of potential virulence factors and related genes in the genome. Detailed comparison results were shown in Table 4.

Table 4 List of potential toxic genes of MNH-5168.

Experimental Example 1

This experimental example studies the effect of the MNC-168 isolated and obtained in Example 1 on macrophages.

Studies in recent years have shown that the gut microorganisms of cancer patients have an important impact on the effect of tumor immunotherapy, and the regulation and utilization of gut microorganisms have gradually become a key part of tumor immunotherapy. It has been demonstrated that multiple mechanisms of direct interaction were existed between gut microorganisms and immune cells, and dendritic cells and macrophages in the gut lymphoid tissue were key target cells for immunomodulatory microorganisms. Especially in the small intestine, because the surface area of the gut was large and the mucus layer was thin and diffuse, allowing microorganisms to come into close contact with immune cells. Different specific strains of the microorganisms can inhibit or activate systemic immune responses.

Macrophages are heterogeneous population of myeloid cells in the innate immune system and participate in various physiological and pathological processes of the body. When inflammation or infection occurs in the body, blood monocytes gather in tissues and differentiate into macrophages. Macrophages have a high plasticity, and mainly have two polarization states, i.e., classically activated type 1 (M1) and alternatively activated type 2 (M2). Mantovani et al. defined these two macrophage phenotypes as extremes of the functional status continuum. M1 macrophages are macrophages that can produce pro-inflammatory cytokines, known as classical macrophages, which often occur after injury and infection. In vitro, macrophages are classically activated by bacterial cell walls such as LPS (lipopolysaccharide) and TNF$\alpha$ (tumor necrosis factor $\alpha$) or IFN$\gamma$ (interferon $\gamma$). M1 macrophages were characterized by secreting proinflammatory factors such as TNF$\alpha$, IL-1$\beta$ (Interleukin-1$\beta$), IL-6 (Interleukin-6), IL-12 (Interleukin-12) and IL-8 (Interleukin-8), which play an important role in the early stage of inflammation. M2 macrophage polarization may be caused by different stimuli, mainly IL-4 (interleukin-4) and/or IL-13 (interleukin-13), and the like. Macrophages activated by IL-4 and IL-13 secrete anti-inflammatory cytokines, such as IL-10 (interleukin-10), CCL18 (chemokine 18) and CCL22 (chemokine 22), which play a role in inhibiting inflammatory response and tissue repair.

M1 macrophages are cytotoxic to both pathogens and tumor cells. Their antitumor activity is related to their ability of secreting reactive nitro groups, reactive oxygen species, and pro-inflammatory cytokines. M2 macrophages promote the growth and survival of tumor cells by secreting many growth factors such as EGF, TGF-$\beta$ or VEGF. Therefore, the detection of macrophage-associated pro-inflammatory factors induced by different strains and the detection of the expression of macrophage-related pro-inflammatory factors and anti-inflammatory factors induced by different strains can be used as a judgment index to judge whether the strain has potential anti-tumor characteristics.

Due to the troublesome isolation process of macrophages, phorbol 12-myrwastate 13-acetate (PMA, Sigma, P8139) was commonly used to induce THP-1 (THP-1 was a human nucleocytic leukemia cell line). Due to their similar reactivity with PBMC (human peripheral blood mononuclear cell)-derived monocyte, it was widely used as a model for studying the immune response ability of monocytes or monocyte-derived macrophages. M0 macrophage, which was differentiated from a monocyte lineage, serve as an in vitro cell model for studying macrophage function. The macrophage model has advantages of easy acquisition, differentiation and polarization.

PMA was added to the cultured THP-1 cell line (Wuhan Punosai Life Technology Co., Ltd.) to a final concentration of 5 ng/ml, further cultured for 48 hours at 37 °C, 5% $CO_2$. Whether M0 cell induced successfully was determined based on the following two standards:

(1) Whether the cells were adherent; and

(2) Expressions of CD14 (lipopolysaccharide receptor) and CD68 (cytoplasmic glycoprotein) were detected by flow cytometry to confirm whether the induction was successful (down-regulation of CD14 expression).

M0 macrophages successfully induced were collected and added with IFN$\gamma$ with a final concentration of 20ng/ml + LPS with a final concentration of 10pg/ml, induced for 24 hours to obtain M1 type macrophages; and M0 macrophages successfully induced were collected and added with IL-4 with a final concentration of 20ng/ml + IL-13 with a final concentration of 20ng/ml, induced for 24 hours to obtain M2 type macrophages.

First, that MNC-168 induces the differentiation of M0 type macrophages into M1 or M2 type cells was observed in this experimental example. The experimental scheme was as follows:
The THP-1 cell line was treated with PMA at a final concentration of 5 ng/ml for 48 hr, and induced to M0 macrophages. A graph showing CD14 expression in the THP1 cell line that is M0 type macrophages induced by PMA detected by flow cytometry is shown in Figure 15.

M0 macrophages were collected and added with IFN$\gamma$ with a final concentration of 20ng/ml + LPS with a final concentration of 10pg/ml, induced for 24 hours to obtain M1 type macrophages.

Secondly, that MNC-168 induces the differentiation of M0 macrophages into M1 cells was observed in this experimental example.

The experimental scheme was as follows:
The THP-1 cell line (commercially purchased from Wuhan Punuosai Life Technology Co., Ltd.) was treated with PMA at a final concentration of 5 ng/ml for 48 h, and differentiated into M0 macrophages.

M0 macrophages were co-incubated with MNC-168 for 24 hr. MNC-168 was added according to the ratio of a MOI (number of viable bacteria: number of cells) = 10:1.Meanwhile, M1 macrophages were used as the control group and M2 macrophages were used as the control group. After anaerobic culture for 1 h, the combined antibiotics listed in Table 5 below were added to kill the bacteria.

Table 5: List of antibiotics used.

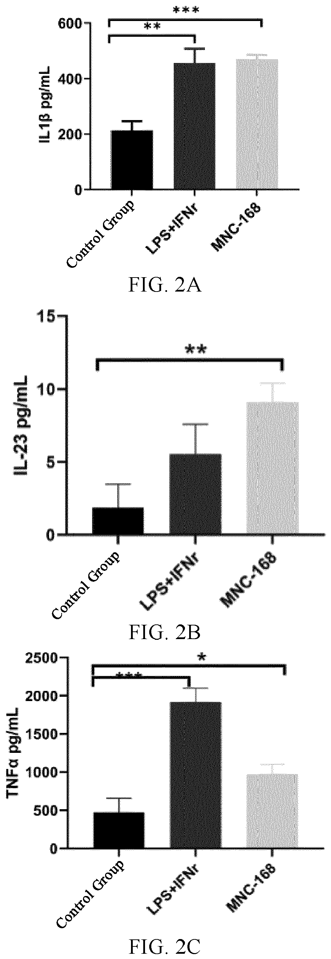

The above culture was incubated at 37 °C, 5% $CO_2$ for 24 h, and the supernatant was collected to detect the concentration of secreting cytokines by ELISA (see Figure 2A, Figure 2B and Figure 2C for the results).

The results showed that after the co-culture of macrophages with MNC-168, the macrophages obviously showed the characteristics of differentiation into M1 macrophages. Among them, the secretion of IL-1$\beta$, IL-23, and TNF$\alpha$ all increased significantly, and the secretion of IL-27 increased to a certain extent, but the increase in the secretion level of IL-27 was lower than that of the other three cytokines.

Experimental Example 2

This experimental example studies the effect of MNC-168 on Primary PBMCs (human primary peripheral blood mononuclear cells).

Studies have shown that the composition of gut microorganisms has a profound impact on the surrounding immune system. Meanwhile, gut microorganisms also have an important impact on the effect of immunotherapy for tumor patients, and are a key part of anti-tumor immunotherapy. Gut microorganisms can interact with immune cells to jointly regulate the immune system. Gut strains can induce primary PBMC cells to differentiate into different cell types and secrete different inflammatory factors, thus they can be used for screening of immune regulation strains.

The purpose of this experiment is to observe the differentiation of Primary PBMC induced by MNC-168. The experimental scheme was as follows:

Commercialized Primary PBMCs (purchased from TPCS, batch number A19Z289100) were cultured in PRMI1640 complete medium (10% heat-inactivated FBS, containing 1% L-glutamine, 0.1% ps (a penicillin-streptomycin mixture), 10mg/ml DNase, the role of DNase was to avoid agglutination) after thawing.

Bacteria were added according to the ratio of a MOI (number of viable bacteria: number of cells) = 1:1, MNC-168 and Primary PBMCs were co-incubated, and PBS control group (PBS and Primary PBMC) was set to co-culture for 24 hours. After anaerobic culture for 1h, the antibiotic combinations listed in Table 6 below were added to kill the bacteria.

Table 6: List of antibiotics used.

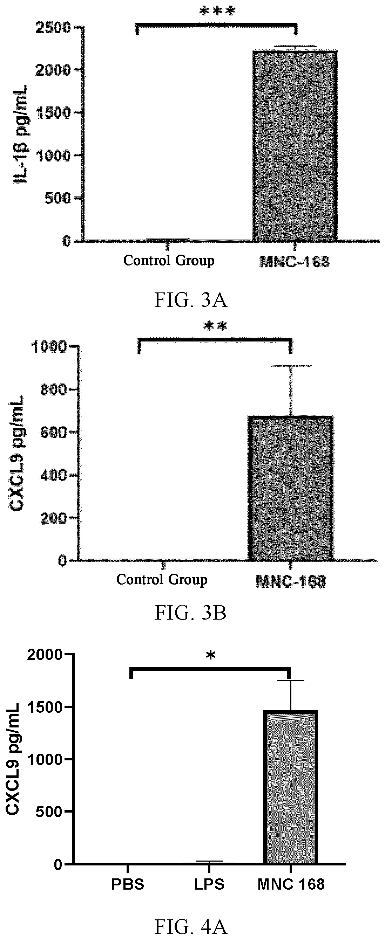

The above culture was incubated at 37 °C, 5% $CO_2$ for 24 hours, and the supernatant was collected to detect the concentration of secreted cytokines by ELISA. The experimental results are shown in Figure 3A and Figure 3B.

The results showed that after the co-culture of primary PBMCs and MNC-168 strain, MNC-168 could induce a significant increase in the inflammatory factors IL1β and CXCL9. The secretion of IFNγ and TNFα also increased to a certain extent, but the increase in the secretion level of IFNγ and TNFα was lower than that of the cytokines IL1β and CXCL9.

Experimental Example 3

This experimental example studies the effect of strain MNC-168 on DC cells.

Gut microorganisms interact with immune cells to jointly regulate the immune system. Gut strains can induce iDC (immature DC, immature dendritic cells) cells to differentiate and mature, and mature DC can effectively activate T cells, thereby killing tumor cells. Therefore, the induced differentiation of iDCs can be used for the screening of immune regulation strains.

Commercialized Primary PBMCs (purchased from TPCS, batch number A19Z289100) were were cultured in PRMI1640 complete medium (10% heat-inactivated FBS, containing 1% L-glutamine, 0.1% ps (a penicillin-streptomycin mixture), 10mg/ml DNase, the role of DNase was to avoid agglutination) after thawing. After 5-6 hours, GM-CSF at a final concentration of 50 ng/ml and IL-4 cytokine at a final concentration of 20 ng/ml were added to the culture to induce iDCs for cultivation for further 5 days.

Effect of MNC-168 on iDC cells.

The purpose of this experiment was to observe the differentiation of iDC cells into mature DC cells induced by MNC-168. The experimental scheme was as follows:

Primary PBMCs were thawed, cultured at 37 °C, 5% $CO_2$ for 5-6 hours, and GM-CSF at a final concentration of 50ng/ml and IL-4 cytokines at a final concentration of 20ng/ml were added to the culture to induce iDCs for cultivation for further 5 days.

Successfully induced iDC cells were co-incubated with MNC-168 for 24hr. Bacteria were added according to the ratio of a MOI (number of viable bacteria: number of cells)=10. Meanwhile, blank control group (PBS) and positive control group (LPS) were used. After anaerobic culture for 1h, the antibiotic combinations listed in Table 7 below were added to kill the bacteria.

Table 7: List of antibiotics used.

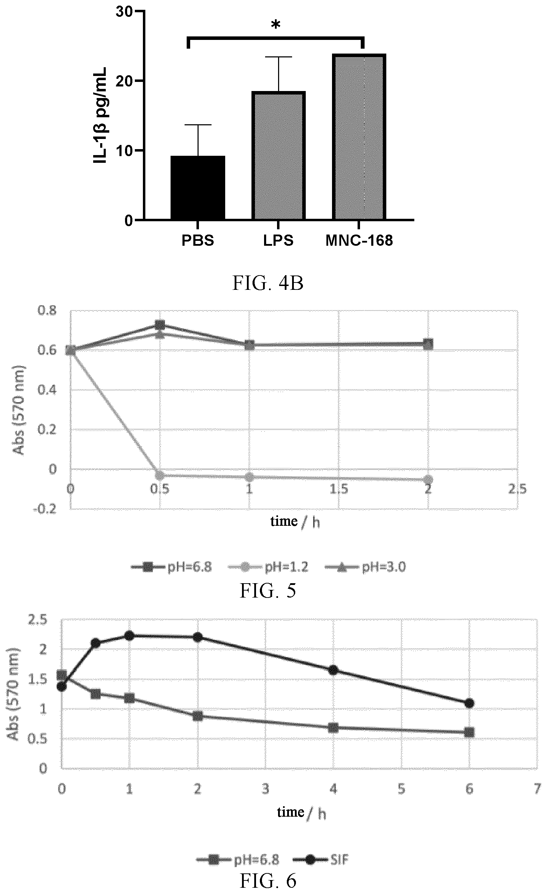

The above culture was incubated at 37 °C, 5% $CO_2$ for 24 hours, and the supernatant was collected to detect the concentration of secreted cytokines by ELISA. The experimental results are shown in Figure 4A and Figure 4B.

The results showed that after the co-culture of iDC cells and MNC-168, the iDC cells clearly displayed the characteristics of differentiation into mature DC cells. Among them, the IL-1β and CXCL9 secreted by iDC cells increased significantly, and the secretion of IFNγ and TRAIL also increased to a certain extent, but the increase in the secretion of IFNγ and TRAIL was not as obvious as that of the other two cytokines.

Experimental Example 4

This experimental example provides the preparation method of MNC-168.

A sterilized toothpick was used to pick a MNC-168 monoclonal into 10mL AC liquid medium (peptone, 20g; glucose, 5g; yeast extract, 3g; beef extract powder, 3g; vitamin C, 0.2g contained per liter; pH 7.0) for 1 day, and 1mL culture solution was sampled for mass spectrometry identification. After the identification result was correct, 2mL bacterial suspension was transferred into 80mL AC liquid medium for cultivation on an anaerobic operating platform.

After culturing for 24 hours, 30mL bacterial suspension was transferred into 400mL AC liquid medium for cultivation on an anaerobic operating platform. After culturing for 24 hours, 1mL bacterial liquid was sampled for mass spectrometry identification. After the identification result was correct, all the bacterial liquid was transferred to a 1L centrifuge bottle and centrifuged at 6000rpm for 30min at 4 °C. The precipitate was resuspended with AC liquid medium: sterile glycerol (4:1).

0.1mL bacterial strain freezing solution was sampled from the bacterial liquid glycerin mixture and added to 0.9mL physiological saline for gradient dilution, diluted to $10^{-8}$ gradient. 100μl of dilution solutions with concentrations of $10^{-6}$, $10^{-7}$, and $10^{-8}$ gradients was added to the anaerobic blood plate medium, respectively. The glass beads were added and shaken well, and CFU thereof was measured by plate counting method.

The prepared strain freezing solution was dispensed to sterile frozen tubes, each tube was filled with 0.2ml bacterial liquid, and each strain was dispensed to 10 tubes, which were stored frozen at -80 °C. After being completely frozen (24h), a tube of frozen bacteria was thawed to room temperature, and the concentration of viable bacteria (CFU) was measured by the dilution coating method.

Experimental Example 5

This experimental example studies the stress resistance of MNC-168.

The development of live biotherapeutic products based on live bacteria has gained more and more attention in recent years. Bacteria will face a very harsh living environment after entering the digestive tract. On the one hand, the low pH gastric acid in the stomach and a certain concentration of bile salts in the gut tract were very harmful to many bacteria. On the other hand, the resistance of different strains to these harmful environments also varies widely.

The resistance of a bacterium to the digestive tract environment is related to whether it has the opportunity to be developed into live biotherapeutic products. These indicators are also meaningful for the selection of formulations in the development of live biotherapeutic products based on the bacterial strain. Therefore, it is necessary for testing the acid resistance and bile salt resistance of the strain.

The way adopted in vitro stress resistance test was to observe the bacteria in the simulated fluid similar to in vivo environment, i.e., simulated gastric fluid (SGF) and simulated intestinal fluid (SIF). The strain was inoculated. Time point for sampling is designed according to the residence time of the strain in the stomach and small intestine, and the survival curve of the strain was determined. In order to exclude the influence of the growth/death of the strain under natural conditions, we used a buffer solution with pH=6.8 as a control. Fasting simulated gastric fluid (SGF) is prepared by a solution at a pH of 1.0 (Chinese Pharmacopoeia) or 1.2 (US Pharmacopoeia) using a concentrated hydrochloric acid dilution method with the addition of pepsin. Considering the changes in gastric fluid before and after meals (referred to the article published by Margareth RC Marques et al.,) we used a pH = 3 as the pH of gastric fluid after meals, which was also similar to the normal pH of gastric fluid in mice. The simulated intestinal fluid (SIF, pH=6.8) was formulated with potassium dihydrogen phosphate and sodium hydroxide in a certain ratio according to USP26.

The duration of the test refers to the emptying time of the human gastrointestinal. For the acid resistance test, we selected 2 hours as the test duration, and for the bile salt resistance test, we selected 6 hours as the test duration. In this experiment, enzymes, inorganic salts, and bile salts were mainly considered. Among them, the activities of pepsin and trypsin on the market were uneven due to different sources and processes. We referred to the literatures published by M.Minekus et al., and Leyuan Li et al., and determined the configuration and protocol of the medium used in the final experiment.

Since the time point of test was quite dense, the operation time of the method of MRS coating was long, and the influence factors are various, it was difficult to guarantee the accuracy of the results. Therefore, in this experiment, the method of MTT (thiazolium blue) staining was used to identify live bacteria. As a dye, MTT is often used as a substrate to detect cell death by detecting the activity of cell succinate dehydrogenase. Succinate dehydrogenase is one of the hinges connecting oxidative phosphorylation and electron transport, providing electrons for the oxygen-demanding and energy-producing respiratory chains of eukaryotic mitochondria and various prokaryotic cells.

Succinate dehydrogenase can reduce exogenous MTT to water-insoluble blue-purple crystalline formazan and deposit it in cells, while dead cells do not have this function. Within a certain range of cell numbers, the amount of formazan crystals formed was in proportion to the number of viable cells. Dimethyl sulfoxide (DMSO) could dissolve formazan precipitated in cells. The light absorption value of dimethyl sulfoxide was measured at a wavelength of 570nm, which could indirectly measure the number of living cells. This method can avoid the tedious operations of concentration dilution and plate coating, which is very suitable for rapid determination of bacterial viability.

I. Simulated Gastric Fluid (SGF) experiment, the experimental protocol comprised:

1. 2.0g NaCl and 2.67g pepsin were weighed (source leaf biological pepsin, USP grade, 1:3000);

2. 7.0mL concentrated HCl (37%) was added and diluted to 1000mL with pure water, and mixed well;

3. The solution was adjusted for pH to 1.2 (pH of simulated human fasting gastric fluid) and pH 3.0 (pH of simulated human gastric fluid after meals and pH of mouse fasting gastric fluid), respectively, filtered and sterilized to obtain the simulated gastric fluid, which was used in the following acid resistance test.

II. Simulated intestinal fluid (SIF) experiment, the experimental protocol comprised:

1. 6.8g potassium dihydrogen phosphate and 0.9g sodium hydroxide were weighed into 1000mL pure water;

2. 0.1g trypsin (source leaf biological trypsin, 1:2500) was added, making the solution reach the enzyme activity of 250U/mL;

3. The solution was adjusted for pH to $6.8\pm0.1$, filtered and sterilized to obtain the simulated intestinal fluid, which was used in the following bile salt resistance test.

III. Preparation of buffer
6.8g of potassium dihydrogen phosphate and 0.9g of sodium hydroxide were dissolved in 1000mL pure water, and adjusted for pH to $6.8\pm0.1$, which was as the buffer used in the following acid resistance test and bile salt resistance test (buffer capacity 18.6mmol /L/pH).

IV. Preparation method of MTT (thiazole blue) staining agent comprised:
0.5g MTT (thiazolyl blue, Shanghai Acmec Biochemical Co.ltd, 98%) was weighed into 100mL sterile PBS (phosphate buffered saline solution, pH=7.2), filtered and sterilized after completely dissolved, stored in a brown reagent bottle, and refrigerated in 4 °C refrigerator.

V. Acid resistance test.

1. 0.7796g of the bacteria powder to be tested was added into 10mL buffer solution to prepare a bacterial solution with a concentration of $2\times10^9$ CFU/mL.

2. 9mL buffer solution (control group), 9mL simulated gastric fluid with a pH of 1.2 (experimental group 1), and 9mL simulated gastric fluid with a pH of 3.0 (experimental group 2) were prepared respectively into centrifuge tubes;

3. 1 mL sample in step 1 of this test was added to the simulated gastric fluid and buffer solution prepared in step 2 of this test, shaked well to obtain samples A ( buffer solution group), B (simulated gastric fluid group with a pH of 1.2), and C (simulated gastric fluid group with a pH of 3.0).

4. The samples were sampled at the sampling time point: sample A was sampled at the time point of 0h (as 0h data); and samples A, B, and C were sampled at 0.5h, 1h and 2h.

5. 1mL samples A, B and C were added to 4mL buffer solution, mixed well, and 3mL suspension was sampled and added to 1mL MTT (5mg/mL) staining solution. The mixture was reacted overnight in a dark environment. 3mL buffer was added to 1mL MTT as a blank control.

6. The mixture was shook well overnight. 1 mL each reaction sample was added to 2 mL dimethyl sulfoxide (DMSO) to dissolve the chromogenic substance formazan.

7. The absorbance value of the sample was measured at a wavelength of 570nM, and the proportion of live bacteria was calculated accordingly.

VI. Bile salt resistance test (conducted at room temperature and in a clean platform).

1. 2 mL bacteria solution to be tested was added to a centrifuge tube containing 18 mL buffer (buffer control group), and 2 mL bacteria solution to be tested was added to a centrifuge tube containing 18 mL simulated intestinal fluid (simulated intestinal fluid group).

2. Sampling time points: 0h (sampled immediately after adding the bacterial solution and mixing), 0.5h, 1h, 2h, 4h, and 6h.

3. 3mL solution of control group and the simulated intestinal fluid group was sampled at the sampling time point and was added to 1mL MTT (5mg/mL) staining solution, and reacted overnight in a dark environment.

4. The mixture was shook well overnight. 1mL each reaction sample was added to 5mL dimethyl sulfoxide (DMSO) to dissolve the chromogenic substance formazan, and then 1mL above sample solution was added into 3mL DMSO for second dilution.

5. The absorbance value of the sample was measured at a wavelength of 570nM, and the proportion of live bacteria was calculated accordingly.

VII. The acid resistance test results are shown in Figure 5, which shows the survival curve of MNC-168 in simulated gastric fluid with reference to absorbance value.

Based on the value of the control group, the survival rate of the living bacteria in the experimental group relative to the control group is shown in Table 8. The result showed that the treatment of simulated gastric fluid at pH 3.0 for 2 hours had little effect on the survival of MNC-168. However, the strain could not resist the simulated gastric fluid at a pH of 1.2 (almost all bacteria died after 0.5 hours of treatment).

Table 8: Survival rate (%) of MNC-168 in simulated gastric fluid.

VIII. Bile salt resistance test results.

The survival curve of MNC-168 in the simulated intestinal fluid with reference to absorbance value is shown in Figure 6.

Based on the value of the control group, the survival rate of live bacteria in the experimental group relative to the control group is shown in Table 9. The result showed that MNC-168 adapted well to the gut fluid environment and proliferated to a certain degree, but the survival rate of MNC-168 decreased slightly over the treatment time.

Table 9: Survival rate (%) of MNC-168 in simulated intestinal fluid.

Experimental Example 6

This experimental example studies whether oral administration of MNC-168 can be used for the treatment of colorectal cancer.

In order to verify whether MNC-168 can be used for the prevention and treatment of tumors, we used a mouse syngeneic tumor model to conduct an experiment of inhibiting the growth of colorectal cancer. This experiment was reviewed for the ethics by the Moon Bioanimal Ethics Committee.

C57B/6 mice were purchased from Nanjing Model Animal Center, and mouse colorectal cancer cells CT26 were purchased from ATCC, InVivoMAb anti-mouse PD-1 (BioXcell, BE0146, specifically anti-mPD-1(CD279) Clone RMP1-14).

Mice were fed with MNC-168 bacteria in p.o.mode. MNC-168 was thawed and placed at room temperature until its temperature reached room temperature, and then fed at a dose of $2 \times 10^9$ CFU (colony formation unit)/day per mouse for 28 consecutive days.

After feeding MNC-168 for seven days, CT26 was vaccinated with the number of cells of $1 \times 10^6$/mouse. The vaccination steps were as follows: CT26 cells were collected on the day of vaccination, the cells were washed once with PBS, resuspended with PBS, and the cell suspension was counted. The cell was adjusted for concentration to $1 \times 10^7$/mL, mixed with precooled Matrigel (basement membrane matrigel) in 1:1 and injected subcutaneously into the mouse at 0.1 mL/mouse. The day of tumor vaccination was D0.

After tumor vaccination, mice were further fed with MNC-168 also in p.o. mode until the end of the experiment.

On the D7, D10, D14, and D17 after tumor vaccination, anti-mouse PD-1 antibody was injected intraperitoneally with the injection dose of 100 μg per mouse.

The size of the tumor was measured every two days, and the volume of the tumor was calculated by the following formula:

$$\text{Tumor volume} = 1/2 \text{ x (long diameter of tumor)}^2 \text{ x wide diameter of tumor.}$$

In addition to the experimental group (MNC-168+PD-1), two groups of controls were also set in the experiment, i.e., PD-1 treatment group (PD-1) and control group without any treatment (Control), respectively.

The experiment was carried out in four groups:

Experimental group 1 (MNC-168+PD-1): MNC-168 was fed and PD-1 antibody was injected according to the method in Experimental Example 6;

Experimental group 2 (MNC-168 treatment group): MNC-168 was fed according to the method in Experimental Example 6;

PD-1 treatment group (PD-1): only PD-1 antibody was injected according to the method in Experimental Example 6;

Control group: no any treatment.

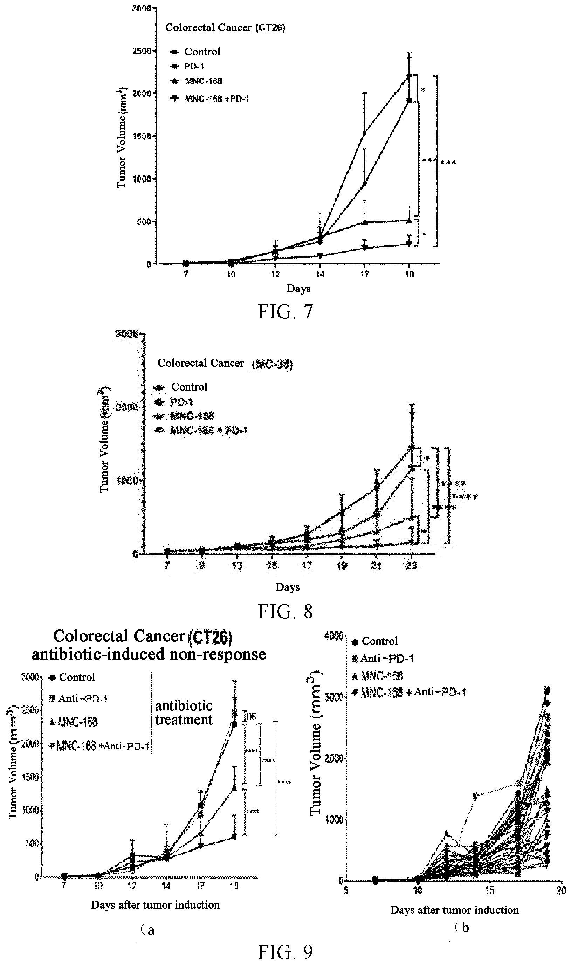

The results are shown in Figure 7. The tumor in the non-treatment group (i.e. the control group) grew rapidly and reached about 2300mm$^3$ on D19, and the tumor growth rate in the PD-1 treatment group was slightly lower than that in the control group. The tumor volume in PD-1 treatment group (PD-1) was about 1800mm$^3$ on the D19, and the tumor volume in MNC-168 treatment group (ie, experimental group 2) was about 500mm$^3$ on the D19, showing that the growth rate was significantly slower than that of the control group and the PD-1 treatment group (PD-1). The results showed that MNC-168 could significantly reduce the growth rate of colorectal cancer even it was administered alone.

The tumor volume in combined treatment group of MNC-168 and PD-1 (i.e. experimental group 1 (MNC-168+PD-1)) was about 200 mm$^3$ on D19, showing that the growth rate was significantly slower than those of the control group and PD-1 treatment group (PD-1). This experiment proved that MNC-168 could significantly reduce the growth rate of colorectal cancer and MNC-168 could significantly reduce the growth rate of colorectal cancer in the presence of PD-1.

Experimental Example 7

In order to verify whether MNC-168 can be used for the prevention and treatment of tumors, we used a mouse syngeneic tumor model to conduct an experiment of inhibiting the growth of colorectal cancer. This experiment was reviewed for the ethics by the Moon Bioanimal Ethics Committee.

C57B/6 mice were purchased from Nanjing Model Animal Center, and mouse colorectal cancer cell MC-38 was purchased from ATCC, InVivoMAb anti-mouse PD-1 (BioXcell, BE0146).

Mice were fed with MNC-168 bacteria in p.o. mode. MNC-168 was thawed and placed at room temperature until its temperature reached room temperature, and then fed at a dose of $2\times10^9$ CFU (colony formation unit)/ day per mouse for 28 consecutive days.

After feeding MNC-168 for seven days, MC38 cells was vaccinated with the number of cells of $2\times10^6$/mouse. The vaccination steps were as follows: MC38 cells were collected on the day of vaccination, the cells were washed once with PBS, resuspended with PBS, and the cell suspension was counted. The cell was adjusted for concentration to $2\times10^7$/mL, mixed with precooled Matrigel in 1:1 and injected subcutaneously into the mouse at 0.1 mL/mouse. The day of tumor vaccination was D0.

After tumor vaccination, mice were further fed with MNC-168 also in p.o. mode until the end of the experiment.

On the D7, D10, D14, and D17 after tumor vaccination, anti-mouse PD-1 antibody was injected intraperitoneally with the injection dose of 200 $\mu$g per mouse.

The size of the tumor was measured every two days, and the volume of the tumor was calculated by the following formula:

$$\text{Tumor volume} = 1/2 \text{ x (long diameter of tumor)}^2 \text{ x wide diameter of tumor.}$$

In addition to the experimental group (MNC-168+PD-1), two groups of controls were also set in the experiment, i.e., PD-1 treatment group (PD-1) only and the control group without any treatment (Control), respectively.

The experiment was carried out in four groups:

Experimental group 1 (MNC-168+PD-1): MNC-168 was fed and PD-1 antibody was injected according to the method in Experimental Example 7;

Experimental group 2 (MNC-168 treatment group): only MNC-168 was fed according to the method of Experimental Example 7;

PD-1 treatment group (PD-1): only PD-1 antibody was injected according to the method of Experimental Example 7;

Blank control group: no treatment.

The results are shown in Figure 8. The tumor in the non-treatment group (i.e. the control group) grew rapidly and reached about 1500 mm$^3$ on D23, and the tumor growth rate in the PD-1 treatment group (PD-1) was slightly lower than that in the control group. The tumor volume in PD-1 treatment group (PD-1) was about 1200mm$^3$ on the D23, and the tumor volume in MNC-168 treatment group (experimental group 2) was about 500mm$^3$ on the D23, showing that the growth rate was slower than those of the control group and the PD-1 treatment group (PD-1). The results showed that MNC-168 alone could significantly reduce the growth rate of colorectal cancer.

The tumor volume in combined treatment group of MNC-168 and PD-1 (experimental group 1) was about 150 mm$^3$ on D23, showing that the growth rate was significantly slower than those of the control group and PD-1 treatment group (PD-1). This experiment proved that MNC-168 could significantly reduce the growth rate of colorectal cancer and MNC-168 could significantly reduce the growth rate of colorectal cancer in the presence of PD-1.

Experimental Example 9

This experimental studies whether oral administration of MNC-168 can increase the response efficiency of anti-PD-1.

In order to verify whether MNC-168 can increase the response efficiency of anti-PD-1, we used a mouse syngeneic tumor models treated with antibiotics to conduct an experiment of inhibiting the growth of colorectal cancer. The list of antibiotics was shown in Table 10. This experiment was reviewed for the ethics by the Moon Bioanimal Ethics Committee.

Table 10: List of antibiotics used.

C57B/6 mice were purchased from Nanjing Model Animal Center, and mouse colorectal cancer cells CT26 were purchased from ATCC, InVivoMAb anti-mouse PD-1 (BioXcell, BE0146).

Mice were fed with MNC-168 bacteria in p.o. mode. MNC-168 was thawed and placed at room temperature until its temperature reached room temperature, and then fed at a dose of $2 \times 10^9$ CFU (colony formation unit)/day per mouse for 28 consecutive days.

After feeding MNC-168 for seven days, CT26 was vaccinated with the number of cells of $1 \times 10^6$/mouse. The steps of vaccination were as follows: the cells were collected on the day of vaccination, the cells were washed once with PBS, resuspended with PBS, and the cell suspension was counted. The cell was adjusted for concentration to $1 \times 10^7$/mL, mixed with precooled Matrigel in 1: 1 and injected subcutaneously into the mouse at 0.1 mL/mouse. The day of tumor vaccination was D0.

After tumor vaccination, mice were further fed with MNC-168 also in p.o. mode until the end of the experiment.

On the D7, D10, D14, and D17 after tumor vaccination, anti-mouse PD-1 antibody was injected intraperitoneally with the injection dose of 100 μg per mouse.

The size of the tumor was measured every two days, and the volume of the tumor was calculated by the following formula:

$$\text{Tumor volume} = 1/2 \text{ x (long diameter of tumor)}^2 \text{ x wide diameter of tumor.}$$

In addition to the experimental group (MNC-168+PD-1), two groups of controls were also set in the experiment, i.e., PD-1 treatment group (PD-1) only and the control group without any treatment (Control), respectively.

The experiment was carried out in four groups:

Experimental group 1 (MNC-168+PD-1): MNC-168 was fed and PD-1 antibody was injected according to the method in Experimental Example 8;

Experimental group 2 (MNC-168 treatment group): MNC-168 was fed according to the method in Experimental Example 8;

PD-1 treatment group (PD-1): only PD-1 antibody was injected according to the method of Experimental Example 8;

Control group: no any treatment.

The results are shown in Figure 9(a). The tumor in the non-treatment group (i.e. the control group) grew rapidly and reached about 2300 mm$^3$ on D19. The tumor growth rate in the PD-1 treatment group was slightly lower than that in the control group. The tumor volume in PD-1 treatment group (PD-1) was about 2500mm$^3$ on the D19, and the tumor volume in MNC-168 treatment group (ie, experimental group 2) was about 1300mm$^3$ on the D19, showing that the growth rate was significantly slower than those of the control group and the PD-1 treatment group (PD-1). The results showed that MNC-168 could significantly reduce the growth rate of colorectal cancer even it was administered alone.

The tumor volume in combined treatment group of MNC-168 and PD-1 (experimental group 1 (MNC-168+PD-1)) was about 600 mm³ on D19, and the growth rate was significantly slower than those of the control group and PD-1 treatment group (PD-1). This experiment proved that MNC-168 could significantly reduce the growth rate of colorectal cancer and MNC-168 could significantly reduce the growth rate of colorectal cancer in the presence of PD-1.

Experimental Example 10

The method of this experimental example comprised:
About 0.3 g of tumor tissue was cut into small pieces with scissors, and digested in collagenase IV digestion solution (1 mg/ml collagenase IV, 0.1 mg/ml DNase, 10% FBS) at 37°C for 30 minutes. The mixture after tissue digestion was screened through a 70um sieve to make a single cell suspension. A certain amount of cell suspension was added to stimulant (100ng/ml PMA, 1ug/ml ionomycin and 6ug/ml BFA were added in 1640 medium), and stimulated at 37°C for 4 hours. After stimulation, staining was carried out with surface molecules CD4 (leukocyte differentiation antigen 4) and CD8 (leukocyte differentiation antigen 8) (FITC Rat Anti-Mouse CD4 (fluorescein isothiocyanate, rat anti-mouse CD4) and PE anti - mouse CD8a (Phycobilichrome (anti-mouse CD8a) were diluted in 1:100 in 1% FBS-PBS solution) at 4 degrees for 30 minutes in the dark. Cells were fixed and ruptured with Transcription Factor Buffer Set, and fixed overnight at 4 degrees with 1X fixative solution, then stained with cytokines (PE-Cy7 anti-mouse IFN-γ was diluted in 1:100 in 1x membrane rupture solution) for 1 hour at room temperature in the dark. Cells were resuspended in PBS and tested on the machine.
Figure 10 shows the flow diagram of tumor-infiltrating immune cells CD4IFNγ cells and CD8IFNγ cells in MNC-168 activated tumor tissues.

Experimental Materials:

FITC Rat Anti-Mouse CD4 (No. 553046, BD), PE Rat Anti-Mouse CD8a (No. 553032, BD).
PE-Cy7 anti-mouse IFN-γ (No. 557649, BD), PMA (P1585, Sigma), ionomycin (No. 407952, Sigma), brefeldin A (No. 00-4506-51, Thermofisher), Transcription Factor Buffer Set (No. 562574, BD), 1640 medium (No.C11875500BT, Thermofisher), FBS (product number 10099-141, Gibico).
The above descriptions are only alternative embodiments of the disclosure, and are not intended to limit the disclosure. For those skilled in the art, the disclosure may have various modifications and changes. Any modifications, equivalent replacements, improvements, etc. that made within the spirit and principles of the disclosure shall be comprised within the protection scope of the disclosure.

Industrial Applicability

Enterococcus lactis of the disclosure has an important impact on the effect of tumor immunotherapy for cancer patients. The Enterococcus lactis can induce the differentiation and maturation of iDC (immature DC) cells, and the mature DCs can effectively activate T cells, thereby killing tumor cells. Meanwhile, the Enterococcus lactis of the disclosure can induce the differentiation of M0 type macrophages into M1 or M2 type cells, resulting in a significant increase in the secretion of IL-1β, IL-23, and TNFα. The Enterococcus lactis of the disclosure can be used in the preparation of tumor inhibitors and corresponding drugs, so as to realize prevention or treatment of the tumor.

**Claims**

1.   Use of Enterococcus lactis in the preparation of a tumor inhibitor, wherein the tumor comprises at least one of the solid tumors from the group consisting of colon cancer, rectal cancer, colorectal cancer, liver cancer, pancreatic cancer, breast cancer, kidney cancer, fibrosarcoma, lung cancer and cholangiocarcinoma.

2.   The use according to claim 1, wherein the Enterococcus lactis is deposited under No. GDMCC NO: 61121 in the Guangdong Microbial Culture Collection Center, named as Enterococcus lactis MNC-168;

     preferably, colony culture characteristics of the Enterococcus lactis are: the colony is white, round, having a moist surface, opaque, and having neat edges after culturing for 24 hours in a bacterial culture medium; and the bacteria are ellipsoid, 0.7-1.0μm×0.8-1.3μm, arranged singly or in pairs under a microscope, and Gram positive;
     preferably, the bacterial culture medium is MRS medium.

3.   The use according to claim 1 or 2, wherein the tumor further comprises the solid tumors from the group consisting of ovarian cancer, cervical cancer, prostate cancer, bladder cancer, head and neck cancer, myeloma, lymphoma, brain tumor, spinal tumor, esophageal cancer, oropharyngeal cancer, laryngeal cancer, colorectal cancer, melanoma, neuroendocrine carcinoma, CNS carcinoma, non-hodgkin lymphoma, hematological malignancies, kidney neoplasms, neuroblastoma, thermostatous sarcoma, Ewing's family sarcoma, retinoma, diffuse large cell lymphoma, and advanced CD70+ cancer.

4. A drug for preventing or treating tumors, wherein the drug comprises Enterococcus lactis.

5. The drug according to claim 4, wherein the drug for preventing or treating tumors further comprises pharmaceutically acceptable additives or adjuvants,

> preferably, the formulation of the pharmaceutical composition is selected from the group consisting of tablet, pill, powder, suspension, gel, emulsion, cream, granule, nanoparticle, capsule, suppositorie, spray and injection; preferably, the Enterococcus lactis is deposited under No. GDMCC NO: 61121 in the Guangdong Microbial Culture Collection Center, named as Enterococcus lactis MNC-168.

6. The drug according to claim 5, wherein the pharmaceutically acceptable additive comprises one or more from the group consisting of filler, diluent, wetting agent, disintegrant, glidant, lubricant, binder, and colourant; and
the pharmaceutically acceptable adjuvant comprises one or more from the group consisting of cyclodextrin, starch, sucrose, lactose, hydroxypropylcellulose, hydroxypropylmethylcellulose, magnesium stearate, talcum, iron oxide, and sodium starch glycolate.

7. A pharmaceutical composition, wherein the pharmaceutical composition comprises the drug for preventing or treating tumors according to any one of claims 4-5;
preferably, the pharmaceutical composition further comprises a drug for use in combination, and the drug for use in combination is at least one of the drugs from the group consisting of chemotherapeutic agent, photosensitizer, photothermal agent, antibody of inhibitory second signaling molecule, inhibitor of inhibitory second signaling molecule, PD-L1 inhibitor, and PD-1/PD-L1 monoclonal antibody.

8. The pharmaceutical composition according to claim 7, wherein the drug for use in combination is at least one of the drugs from the group consisting of chemotherapeutic agent, photosensitizer, photothermal agent, antibody of inhibitory signaling pathway molecule, inhibitor of inhibitory signaling pathway molecule, antibody of inhibitory second signaling pathway molecule, inhibitor of inhibitory second signaling pathway molecule, PD-L1 inhibitor, and PD-1/PD-L1 monoclonal antibody.

9. The pharmaceutical composition according to claim 7, wherein the inhibitor of inhibitory second signaling molecule comprises at least one of the signaling molecules from the group consisting of PD-1 and CTLA-4.

10. The pharmaceutical composition according to claim 8, wherein the inhibitory signaling pathway molecule comprises at least one of the signaling molecules from the group consisting of PD-1 and CTLA-4.

11. The pharmaceutical composition according to claim 9, wherein the antibody of inhibitory second signaling molecule or the inhibitor of inhibitory second signaling molecule comprises at least one of the antibodies from the group consisting of nivolumab, pembrolizumab, tislelizumab, nivolumab injection, pembrolizumab injection, toripalimab injection and sintilimab injection.

12. The pharmaceutical composition according to claim 10, wherein the antibody of inhibitory signaling pathway molecule or the inhibitor of inhibitory signaling pathway molecule comprises at least one of the antibodies from the group consisting of nivolumab, pembrolizumab, tislelizumab, nivolumab injection, pembrolizumab injection, toripalimab injection and sintilimab injection.

13. The pharmaceutical composition according to claim 7 or 8, wherein the PD-L1 inhibitor is selected from durvalumab, atezolizumab or avelumab.

14. The pharmaceutical composition according to claim 7 or 8, wherein the PD-1/PD-L1 monoclonal antibody is selected from pembrolizumab or nivolumab.

15. Enterococcus lactis, wherein the Enterococcus lactis is deposited under No. GDMCC NO: 61121 in the Guangdong Microbial Culture Collection Center, named as Enterococcus lactis MNC-168;

preferably, the colony culture characteristics of Enterococcus lactis are: the colony is white, round, having a moist surface, opaque, and having neat edges after culturing for 24 hours in a bacterial culture medium; and the bacteria is ellipsoid, 0.7-1.0$\mu$m $\times$ 0.8-1.3$\mu$m, arranged singly or in pairs under a microscope, and Gram positive;
preferably, the bacterial culture medium is MRS medium.

16. Use of the drug according to any one of claims 4-6, and/or the pharmaceutical composition according to any one of claims 7-14 for the treatment of tumors.

17. A method for treating tumors, wherein the method comprises:
administering a therapeutically effective amount of the drug according to any one of claims 4-6, and/or the pharmaceutical composition according to any one of claims 7-14 to the subject in need thereof.

18. The drug according to any one of claims 4-6, and/or the pharmaceutical composition according to any one of claims 7-14, and/or the method according to claim 17, wherein the tumor comprises at least one of the solid tumors from the group consisting of colon cancer, rectal cancer, colorectal cancer, liver cancer, pancreatic cancer, breast cancer, kidney cancer, fibrosarcoma, lung cancer, and cholangiocarcinoma.

19. The method according to claim 17, wherein the drug or the pharmaceutical composition is administered via at least one from the group consisting of oral, intravenous, subcutaneous, intraperitoneal, rectal, intramuscular, dermal, transdermal, topical, and via any other parenteral route, in a pharmaceutically acceptable formulation.

FIG. 1

FIG. 2A

FIG. 2B

FIG. 2C

FIG. 3A

FIG. 3B

FIG. 4A

FIG. 4B

FIG. 5

FIG. 6

FIG. 7

FIG. 8

FIG. 9

FIG. 10

FIG. 11

FIG. 12

FIG. 13

FIG. 14

FIG. 15

## INTERNATIONAL SEARCH REPORT

| International application No. |
| --- |
| **PCT/CN2021/098848** |

**A.    CLASSIFICATION OF SUBJECT MATTER**

C12N 1/20(2006.01)i;  C12N 1/02(2006.01)i;  A61K 35/744(2015.01)i;  A61K 45/06(2006.01)i;  A61P 35/00(2006.01)i

According to International Patent Classification (IPC) or to both national classification and IPC

**B.    FIELDS SEARCHED**

Minimum documentation searched (classification system followed by classification symbols)

C12N, A61K

Documentation searched other than minimum documentation to the extent that such documents are included in the fields searched

Electronic data base consulted during the international search (name of data base and, where practicable, search terms used)

数据库: DWPI, SIPOABS, CNABS, USTXT, WOTXT, EPTXT, CNTXT, ISI WEB OF KNOWLEDGE, PUBMED, CNKI 检索词: 癌症, 肿瘤, 乳酸肠球菌, 肠球菌, PD-1, MNC-168, GDMCC NO: 61121, cancer, Enterococcus lactis, Enterococcus lactis

**C.    DOCUMENTS CONSIDERED TO BE RELEVANT**

| Category* | Citation of document, with indication, where appropriate, of the relevant passages | Relevant to claim No. |
| --- | --- | --- |
| PX | CN 112618576 A (MUEN (GUANGZHOU) BIO-TECH CO., LTD.) 09 April 2021 (2021-04-09)<br>    see entire document | 1-16, 18 |
| A | CN 104195075 A (SYNBIO TECH INC.) 10 December 2014 (2014-12-10)<br>    see entire document | 1-16, 18 |
| X | CN 101067120 A (KANGZHE MEDICINE RESEARCH (SHENZHEN) CO., LTD.) 07 November 2007 (2007-11-07)<br>    see entire document<br>    see abstract and page 7 | 1-7, 15, 16, 18 |
| Y | Yousef Nami et al. "Frontiers in Microbiology"<br>*The Prophylactic Effect of Probiotic Enterococcus lactis IW5 against Different Human Cancer Cells*, Vol. 6, 31 December 2015 (2015-12-31),<br>ISSN: 1664-302X,<br>    see abstract and page 7 | 8-14 |

☑ Further documents are listed in the continuation of Box C.    ☑ See patent family annex.

| *  Special categories of cited documents: | "T"  later document published after the international filing date or priority date and not in conflict with the application but cited to understand the principle or theory underlying the invention |
| --- | --- |
| "A"  document defining the general state of the art which is not considered to be of particular relevance | |
| "E"  earlier application or patent but published on or after the international filing date | "X"  document of particular relevance; the claimed invention cannot be considered novel or cannot be considered to involve an inventive step when the document is taken alone |
| "L"  document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O"  document referring to an oral disclosure, use, exhibition or other means | |
| "P"  document published prior to the international filing date but later than the priority date claimed | "&"  document member of the same patent family |

| Date of the actual completion of the international search | Date of mailing of the international search report |
| --- | --- |
| **04 August 2021** | **07 September 2021** |

| Name and mailing address of the ISA/CN | Authorized officer |
| --- | --- |
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2021/098848** |

**C.    DOCUMENTS CONSIDERED TO BE RELEVANT**

| Category* | Citation of document, with indication, where appropriate, of the relevant passages | Relevant to claim No. |
| --- | --- | --- |
| Y | Lisa Derosa et al. "The intestinal microbiota determines the clinical efficacy of immune checkpoint blockers targeting PD-1/PD-L1"<br>*Oncoimmunology*, Vol. 7, No. 6, 31 December 2018 (2018-12-31),<br>ISSN: 2162-402X,<br>      see abstract and table 1 | 8-14 |

## INTERNATIONAL SEARCH REPORT

| International application No. |
|---|
| **PCT/CN2021/098848** |

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

**INTERNATIONAL SEARCH REPORT**

International application No.

**PCT/CN2021/098848**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **17, 19**
   because they relate to subject matter not required to be searched by this Authority, namely:

   [1]   A diagnosis and treatment method for diseases.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2021/098848**

| Patent document cited in search report | | | Publication date (day/month/year) | Patent family member(s) | | | Publication date (day/month/year) |
|---|---|---|---|---|---|---|---|
| CN | 112618576 | A | 09 April 2021 | None | | | |
| CN | 104195075 | A | 10 December 2014 | CN | 104195075 | B | 19 April 2017 |
| CN | 101067120 | A | 07 November 2007 | HK | 1110098 | A1 | 04 July 2008 |
| | | | | CN | 100506974 | C | 01 July 2009 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202110072294
- CN 202110085233